(19) **Europäisches Patentamt European Patent Office Office européen des brevets**

(11) **EP 4 509 162 A1**

(12) **EUROPEAN PATENT APPLICATION**
published in accordance with Art. 153(4) EPC

(43) Date of publication:
**19.02.2025 Bulletin 2025/08**

(21) Application number: **23788025.7**

(22) Date of filing: **21.02.2023**

(51) International Patent Classification (IPC):
*A61M 60/822* (2021.01)    *A61M 60/113* (2021.01)
*A61M 60/117* (2021.01)    *A61M 60/232* (2021.01)
*A61M 60/38* (2021.01)    *A61M 60/419* (2021.01)
*A61M 60/508* (2021.01)    *A61M 60/804* (2021.01)
*F04D 29/048* (2006.01)    *F16C 32/04* (2006.01)

(52) Cooperative Patent Classification (CPC):
**A61M 60/113; A61M 60/117; A61M 60/232;
A61M 60/38; A61M 60/419; A61M 60/508;
A61M 60/804; A61M 60/822; F04D 29/048;
F16C 32/04**

(86) International application number:
**PCT/JP2023/006133**

(87) International publication number:
**WO 2023/199602 (19.10.2023 Gazette 2023/42)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **12.04.2022 JP 2022065596**

(71) Applicants:
• **Institute of Science Tokyo
  Tokyo 152-8550 (JP)**
• **National University Corporation
  Asahikawa Medical University
  Asahikawa, Hokkaido 078-8510 (JP)**

(72) Inventors:
• **HIJIKATA, Wataru
  Tokyo 152-8550 (JP)**

• **HATAKENAKA, Kohei
  Tokyo 152-8550 (JP)**
• **FUJIWARA, Tatsuki
  Tokyo 152-8550 (JP)**
• **OHUCHI, Katsuhiro
  Tokyo 152-8550 (JP)**
• **ARAI, Hirokuni
  Tokyo 152-8550 (JP)**
• **MIZUNO, Tomohiro
  Tokyo 152-8550 (JP)**
• **INOUE, Yusuke
  Asahikawa-shi, Hokkaido 078-8510 (JP)**
• **TAKEWA, Yoshiaki
  Asahikawa-shi, Hokkaido 078-8510 (JP)**

(74) Representative: **SSM Sandmair
Patentanwälte Rechtsanwalt
Partnerschaft mbB
Joseph-Wild-Straße 20
81829 München (DE)**

(54) **DRIVE CONTROL METHOD FOR MAGNETICALLY LEVITATED BLOODFLOW PUMP, DRIVE CONTROL DEVICE FOR MAGNETICALLY LEVITATED BLOODFLOW PUMP, AND MAGNETICALLY LEVITATED BLOODFLOW PUMP SYSTEM**

(57) The purpose of the present invention is to provide a drive control method for a magnetically levitated blood pump, a drive control device for a magnetically levitated blood pump, and a magnetically levitated blood pump system for preventing a formation of thrombus in a housing, and also, for detecting a position of thrombus surely from a phase difference $\triangle \varphi$ between a phase of a waveform of an electromagnet current and a phase of a displacement of an impeller (phase of vibration waveform) without depending on a viscosity $\mu$ and a temperature of blood. In a magnetically levitated blood pump 20 comprising an electric motor 24 for rotating an impeller 23 in a housing 22 by a magnetic coupling force in a Z axis direction in a state that the impeller 23 is magnetically levitated by electromagnets 25X, 25Y for magnetic levitation, the impeller 23 is rotated and revolved in the housing 22 by a drive control device 30 comprising a rotation drive control unit 31 functioning as a rotation

**(Cont. next page)**

EP 4 509 162 A1

drive control means for rotating the impeller 23 and a revolution drive control unit 32 functioning as a revolution drive control means for revolving the impeller 23 by moving the impeller 23 to trace a revolving locus in which a rotation center of the impeller 23 is moved in a radial direction.

FIG.1

## Description

Background of the Invention

Field of the Invention

The present invention relates to a drive control method for a magnetically levitated blood pump, a drive control device for a magnetically levitated blood pump, and a magnetically levitated blood pump system for feeding blood by magnetically levitating an impeller (vane wheel) with a magnetic bearing, which can be used as an artificial heart or the like.

Description of Related Art

A blood pump system for assisting a blood circulation by a blood pump comprising a housing having a blood inlet port and a blood outlet port connected to a blood supply tube, an impeller for feeding blood by rotating in the housing, and an electric motor for rotating the impeller, is widely used as a blood circulation system in an artificial heart and lung apparatus or a heart assisting pump for assisting a heart action.

For example, in a heart surgery, a blood circulation system is used as an extracorporeal blood circulation path for temporally substituting a cardiopulmonary function of a patient. The extracorporeal blood circulation path comprises a blood pump for flowing blood into the path.

Other than heart surgery, a blood circulation system is used as an extracorporeal blood circulation path for a long period of time from few days to few weeks for an assisted circulation for assisting a cardiopulmonary function in a case such as a heart failure or a respiratory failure. When blood is circulated by a blood pump for such long time, a thrombus may be formed in a blood pump or an artificial lung and a blood flow path. When a thrombus is fed into a body of a patient together with blood, serious complications such as embolization of blood vessel may occur.

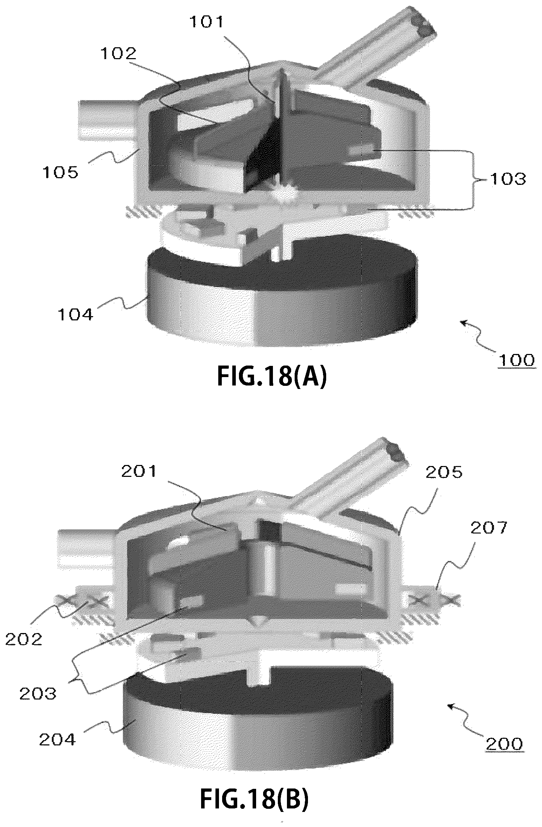

In an intracorporeal implanted blood pump (auxiliary artificial heart) used for assisting a blood circulation of a heart failure patient, conventionally as illustrated in Fig. 18(A), a centrifugal blood pump 100, in which an impeller 102 is supported by using a contact bearing (pivot bearing) 101 and the impeller 102 in a housing 105 is rotated by an electric motor 104 with a magnetic coupling force 103 in a Z direction, was used, but the contact bearing 101 is used without lubrication to avoid a contamination of blood, so there is a severe abrasion and friction, and by a stagnation, a shearing force, and an abrasion of the bearing, there were problems such as a lowering of durability, a destruction of blood cells (hemolysis), and a formation of thrombus occur, so for a purpose of an improvement of durability and a reduction of damage of blood, as illustrated in Fig. 18(B), a magnetically levitated blood pump 200, in which an impeller 201 for discharging blood is magnetically levitated by electromagnets 202 for magnetic levitation without using a mechanical bearing, and the impeller 201 in a housing 205 is rotated by an electric motor 204 with a magnetic coupling force 203 in a Z direction in a completely non-contact state, is becoming a mainstream (for example, refer to Patent Literatures 1, 2 and 3). Further, a magnetically levitated blood pump is becoming to be used for a blood pump for extracorporeal circulation or an extracorporeal membrane oxygenation (ECMO) device which is an extracorporeal circulation type life-support equipment, in which a blood (vein blood) is extracted from a human body and the extracted blood is fed to an artificial lung by using a centrifugal blood pump and a blood oxidized and removed of carbon dioxide in the artificial lung is returned to a vein or an artery.

In the magnetically levitated blood pump, the impeller in the housing is rotated in a completely non-contact state, so problems such as a stagnation, a shearing force, and an abrasion of the bearing can be solved, but a formation of thrombus is still being a problem to be solved.

The present inventors have performed ECMO treatments to many COVID-19 patients, and experienced that patients infected to COVID-19 show extremely strong blood clotting tendencies. Similar reports have been made worldwide.

In ECMO treatment to COVID-19, a large amount of thrombi are formed in a centrifugal blood pump or an artificial lung, and it is difficult to control thrombus even by using a large amount of anticoagulant.

When there is an excess amount of anticoagulant, there is a risk of bleeding complications, so in addition to the anticoagulant, an achievement of a thrombus prevention technology in a circulation path, an artificial lung, and a blood pump is an urgent issue.

In other words, not only auxiliary artificial heart, but also in a blood pump for extracorporeal circulation such as extracorporeal membrane oxygenation (ECMO) device, a thrombus detection and a thrombus prevention technology are required.

The present inventors have reported previously that, in this magnetically levitated blood pump, a thrombus can be detected from a phase difference of a displacement of an impeller and an electromagnet current by reciprocally exciting the impeller in a diameter direction (for example, refer to Non-Patent Literature 1).

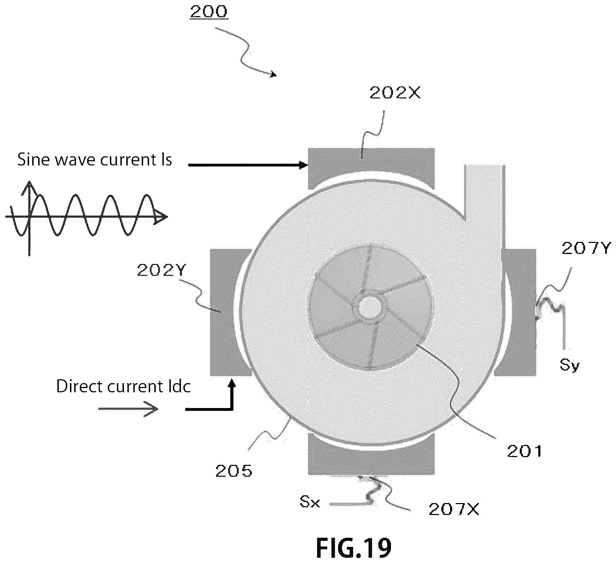

In other words, as illustrated in Fig. 19, in a magnetically levitated blood pump 200, by reciprocally exciting an impeller 201 in a diameter direction by supplying a sine wave current to one of electromagnets 202X for magnetic levitation and electromagnets 202Y for magnetic levitation orthogonally arranged in a diameter direction of the impeller 201 and arranged for magnetically levitating the impeller 201 in a housing 205 to retain a standing posture, an adhesion of thrombocyte can be prevented by a minute vibration of the impeller 201, and a formation of thrombus can be prevented. Also, in the magnetically levitated blood pump 200, by reciprocally exciting the impeller in a diameter (X axis) direction for example by supplying a sine wave current to the electromagnets 202X for magnetic levitation, a vibration waveform according to a reciprocally exciting state of the impeller 201 is detected by a displacement sensor 207X arranged for controlling a magnetic levitation of the impeller 201, and when a thrombus is generated, a phase of a vibration waveform according to a displacement in a diameter direction of the impeller 201 detected by the displacement sensor 207X changes, so a thrombus can be detected from a phase difference $\triangle cp$ between a phase of the sine wave current, i.e. electromagnet current, and a phase of a vibration waveform detected by the displacement sensor 207X.

A phase difference $\triangle\varphi$ between a phase of a waveform of an electromagnet current and a phase of a displacement of the impeller (phase of vibration waveform) is proportional to a blood viscosity $\mu$, and changes inversely proportional to a cube of a gap $g_r$ changing by a formation of thrombus with respect to a most narrow radial gap $g_r$ between the impeller 201 and an inner wall of the housing 205, and having a relation indicated in the following formula (1).

$$\triangle\varphi \propto \mu/g_r^3 \qquad \text{Formula (1)}$$

Patent Literature 1: JP 2009-106690 A
Patent Literature 2: JP 2020-139484 A
Patent Literature 3: WO2010/104031A1

Non-Patent Literature 1: Wataru H, Takuro M, Tomotaka M, Daisuke S, Osamu M, Detection of thrombosis in a magnetically levitated blood pump by vibrational excitation of the impeller. Artificial Organs. 2020;44:594-603.

Summary of the Invention

As described in the above, in a magnetically levitated blood pump for rotating an impeller in a housing in a completely noncontact state, by reciprocally exciting the impeller in a diameter direction, a phase difference $\triangle\varphi$ between a phase of a waveform of an electromagnet current and a phase of a displacement of the impeller (phase of vibration waveform) changes inversely proportional to a cube of a gap $g_r$ changing by a formation of thrombus, so a thrombus can be detected, but this phase difference $\triangle\varphi$ also depends on a viscosity $\mu$ and a temperature of blood, so a distinction from a thrombus was difficult.

Here, considering the above conventional circumstances, a purpose of the present invention is to provide a drive control method for a magnetically levitated blood pump, a drive control device for a magnetically levitated blood pump, and a magnetically levitated blood pump system for preventing a formation of thrombus in a housing, and also, for detecting a position of thrombus surely from a phase difference $\triangle\varphi$ between a phase of a waveform of an electromagnet current and a phase of a displacement of an impeller (phase of vibration waveform) without depending on a viscosity $\mu$ and a temperature of blood.

Other purposes of the present invention and concrete advantages obtained by the present invention will be even more clear from explanations of embodiments explained in below.

In the present invention, by measuring an electrical angle of a driving current and a trajectory of an impeller by exciting the impeller in orbit to rotate and revolve the impeller, it is possible to detect a position of thrombus formed in the pump from its phase difference $\triangle\varphi$, and also, a formation of thrombus in the pump can be prevented by this orbit excitation.

In other words, the present invention is a drive control method for a magnetically levitated blood pump for rotating an impeller in a housing in a state magnetically levitated by electromagnets, wherein the impeller is rotated and revolved by moving the impeller to trace a revolving locus in which a rotation center of the impeller is moved in a radial direction.

In the drive control method for a magnetically levitated blood pump relating to the present invention, a period of revolution of the impeller is an integer multiple of a period of rotation of the impeller or an integer division of a period of rotation of the impeller.

Also, in the drive control method for a magnetically levitated blood pump relating to the present invention, the impeller may be revolved in a circular orbit.

Also, in the drive control method for a magnetically levitated blood pump relating to the present invention, the impeller may be revolved in an elliptical orbit.

Also, in the drive control method for a magnetically levitated blood pump relating to the present invention, the impeller may be revolved to trace a revolving locus rotating an oscillation direction while simple oscillation of a rotation center of the impeller in a radial direction.

Also, in the drive control method for a magnetically levitated blood pump relating to the present invention, the impeller in the housing may be revolved in a circular orbit by supplying a sine wave current and a cosine wave current to electromagnets for magnetic levitation orthogonally arranged in a X axis direction and a Y axis direction orthogonal to a rotation axis direction of the impeller for magnetically levitating the impeller in the housing, while rotating the impeller in the housing by an electric motor with a magnetic coupling force.

Also, in the drive control method for a magnetically levitated blood pump relating to the present invention, the impeller in the housing may be revolved in an elliptical orbit by deviating a phase of one of a sine wave current and a cosine wave current supplied to electromagnets for magnetic levitation orthogonally arranged in a X axis direction and a Y axis direction orthogonal to a rotation axis direction of the impeller for magnetically levitating the impeller in the housing, while rotating the impeller in the housing by an electric motor with a magnetic coupling force.

The present invention is a drive control device for a magnetically levitated blood pump for rotating an impeller in a housing in a state magnetically levitated by electromagnets, comprising: a rotation drive control means for rotating the impeller by an electric motor with a magnetic coupling force; and a revolution drive control means for revolving the impeller by moving the impeller to trace a revolving locus in which a rotation center of the impeller is moved in a radial direction, wherein the impeller is rotated and revolved.

In the drive control device for a magnetically levitated blood pump relating to the present invention, the rotation drive control means and the revolution drive control means may rotate and revolve the impeller in a period of revolution which is an integer multiple of a period of rotation or an integer division of a period of rotation.

Also, in the drive control device for a magnetically levitated blood pump relating to the present invention, the revolution drive control means may revolve the impeller in a circular orbit.

Also, in the drive control device for a magnetically levitated blood pump relating to the present invention, the revolution drive control means may revolve the impeller in an elliptical orbit.

Also, in the drive control device for a magnetically levitated blood pump relating to the present invention, the revolution drive control means may revolve the impeller to trace a revolving locus rotating an oscillation direction while simple oscillation of a rotation center of the impeller in a radial direction.

Also, in the drive control device for a magnetically levitated blood pump relating to the present invention, the rotation drive control means may rotate the impeller in the housing by an electric motor with a magnetic coupling force, and the revolution drive control means may revolve the impeller in the housing in a circular orbit by supplying a sine wave current and a cosine wave current to electromagnets for magnetic levitation orthogonally arranged in a X axis direction and a Y axis direction orthogonal to a rotation axis direction of the impeller for magnetically levitating the impeller in the housing.

Also, in the drive control device for a magnetically levitated blood pump relating to the present invention, the revolution drive control means may revolve the impeller in the housing in an elliptical orbit by deviating a phase of one of the sine wave current and the cosine wave current supplied to the electromagnets for magnetically levitating the impeller in the housing.

The present invention is a magnetically levitated blood pump system, comprising: a drive control device of a magnetically levitated blood pump relating to any of the present inventions; and the magnetically levitated blood pump which is drive controlled by the drive control device, wherein an impeller of the magnetically levitated blood pump is rotated and revolved.

The magnetically levitated blood pump system of the present invention may comprise a computing means for calculating a phase difference of a locus of displacement of the impeller when the impeller is revolved and a locus of current of electromagnets for revolving the impeller.

In the present invention, by rotating and revolving the impeller by exciting the impeller in orbit, in a phase difference $\triangle\varphi$ between a phase of a drive current waveform for revolving the impeller and a phase of a displacement of a rotation center of the impeller by a revolution of the impeller (phase of vibration waveform), a change due to a viscosity $\mu$ and a temperature of blood will be a direct current component in a period of revolution, so it can be distinguished surely from a change of the phase difference $\triangle$cp due to a formation of thrombus.

In the present invention, by measuring an electrical angle of a driving current and a trajectory of an impeller by exciting the impeller in orbit to rotate and revolve the impeller, and not by a known reciprocal excitation of impeller, it is possible to detect a position of thrombus formed in the pump from its phase difference $\triangle\varphi$, and also, a formation of thrombus in the pump can be prevented by this orbit excitation.

Also, in the present invention, by rotating and revolving the impeller in a period of revolution which is an integer multiple of a period of rotation or an integer division of a period of rotation, a thrombus formed at an inner wall of the housing and a thrombus formed at an outer wall of the impeller can be detected and distinguished from the phase difference.

Also, in the present invention, by revolving the impeller in an elliptical orbit, a thrombus formed in the pump can be detected from the phase difference without influenced by a machining error of an inner wall of the housing.

Therefore, according to the present invention, it is possible to provide a drive control method for a magnetically levitated blood pump, a drive control device for a magnetically levitated blood pump, and a magnetically levitated blood pump system for preventing a formation of thrombus in a housing, and also, for detecting a position of thrombus surely from a phase difference $\triangle\varphi$ between a phase of a waveform of an electromagnet current and a phase of a displacement of the impeller (phase of vibration waveform) without depending on a viscosity $\mu$ and a temperature of blood.

Brief Description of the Drawings

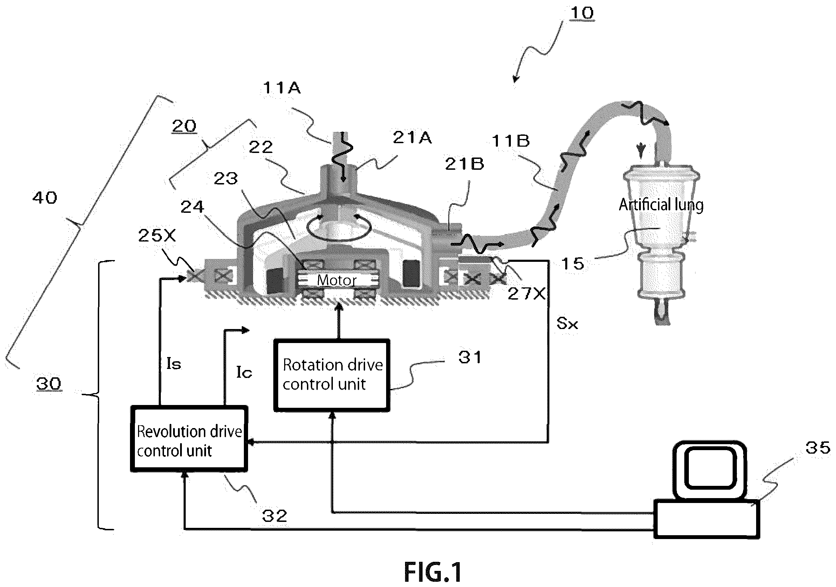

Fig. 1 is a schematic view illustrating a structure of an extracorporeal blood circulation system in which the present invention is performed.

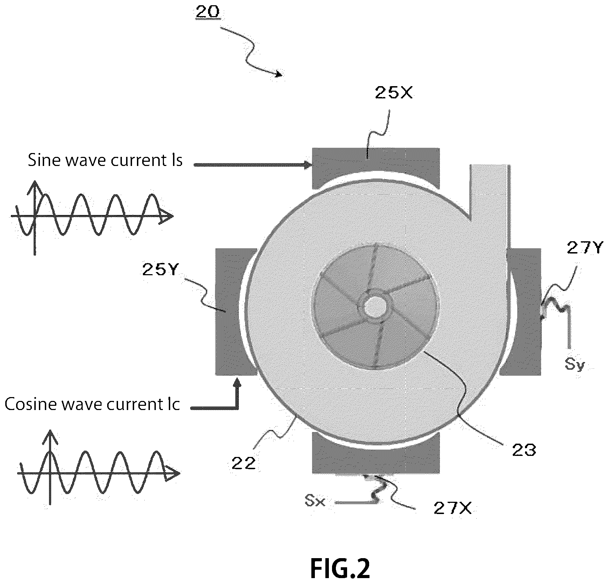

Fig. 2 is a schematic plan view of a magnetically levitated blood pump schematically illustrating a supply state of a revolution drive control current by a revolution drive control unit functioning as a revolution drive control means for revolving an impeller in a housing of the magnetically levitated blood pump in a circular orbit, in a drive control device of a magnetically levitated blood pump system.

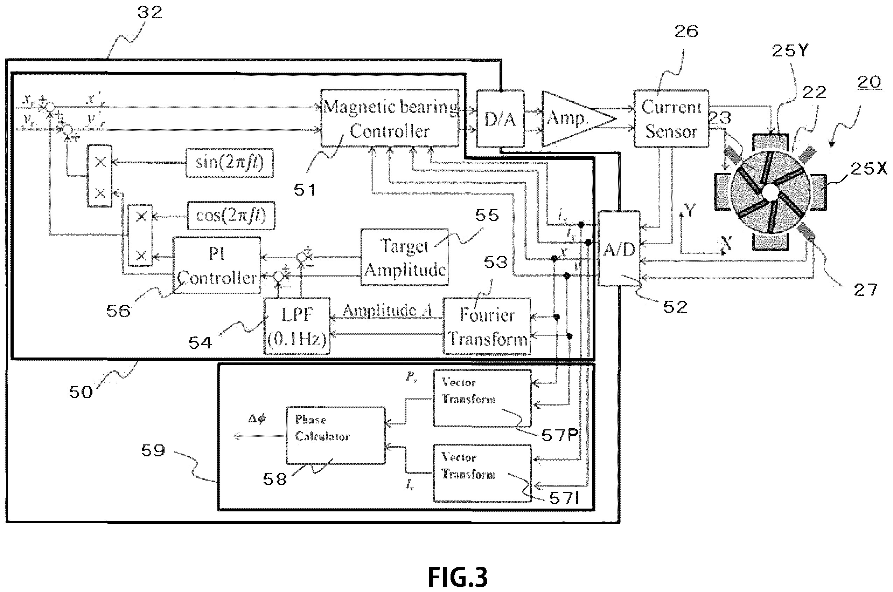

Fig. 3 is a block diagram illustrating a structural example of the revolution drive control unit.

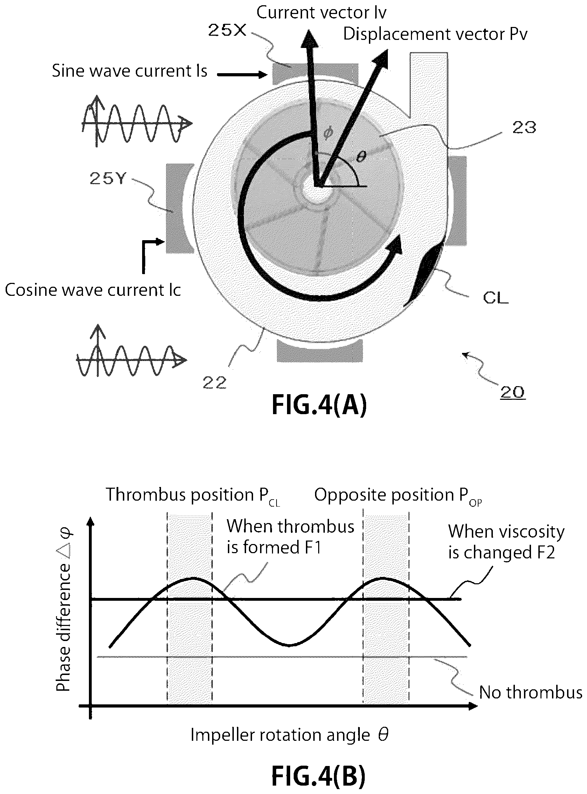

Fig. 4(A) and Fig. 4(B) are views for explaining a detection of thrombus by an excitation in a circular orbit of an impeller in the magnetically levitated blood pump, and Fig. 4(A) is a schematic plan view of the magnetically levitated blood pump illustrating a relative angle between a displacement vector of the impeller according to a revolution of the impeller and a current vector according to a revolution of the impeller, i.e. a phase of a drive current for revolving the impeller in a circular orbit, and Fig. 4(B) is a characteristic diagram of a phase difference illustrating a change of a phase difference by a formation of thrombus and a change of a phase difference by a change of viscosity obtained by measuring the relative angle between the displacement vector of the impeller and the current vector as a phase difference between a phase of the drive current and a phase of a rotation of the impeller, as a change according to a rotation angle of the impeller.

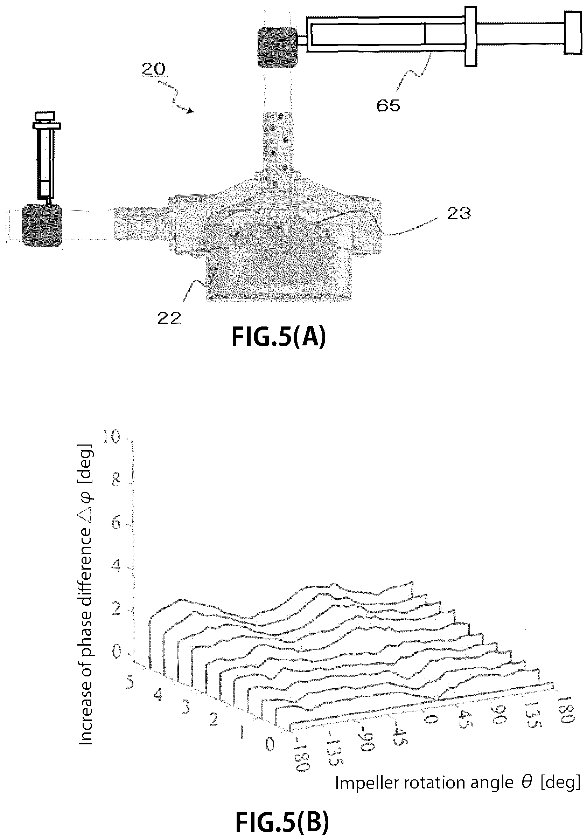

Fig. 5(A) and Fig. 5(B) are views for explaining a change of a phase difference when a viscosity is changed in the magnetically levitated blood pump system, and Fig. 5(A) is a view schematically illustrating a structure of an experimental equipment for measuring a change of a phase difference when a viscosity is changed, and Fig. 5(B) is a characteristic diagram illustrating a change of measured phase difference.

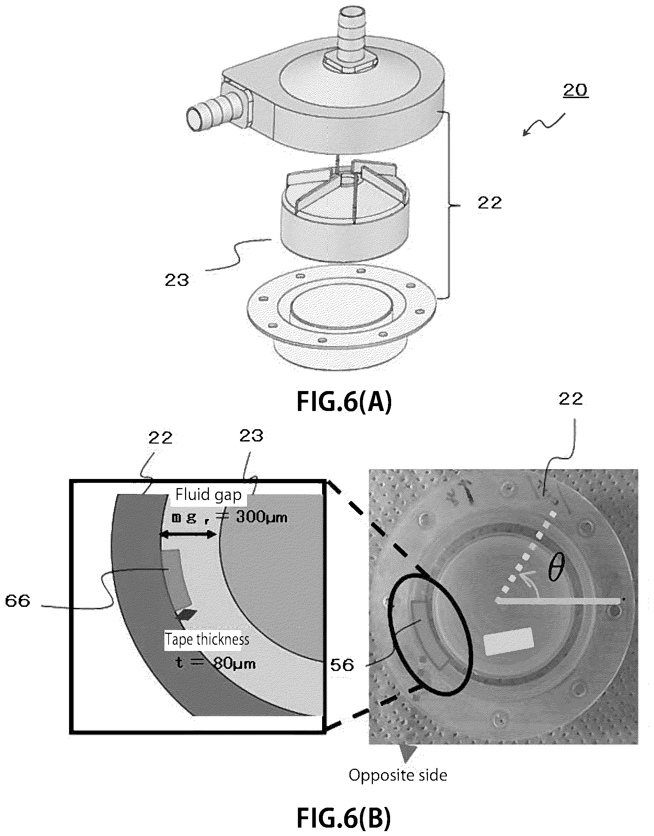

Fig. 6(A) and Fig. 6(B) are views for explaining a change of a phase difference by a simulated thrombus (tape) about a detection of thrombus in the magnetically levitated blood pump system, and Fig. 6(A) is an exploded perspective view of a housing of the magnetically levitated blood pump, and Fig. 6(B) is a view illustrating a state that a tape is adhered as a substitution of a thrombus at an inner wall of the housing.

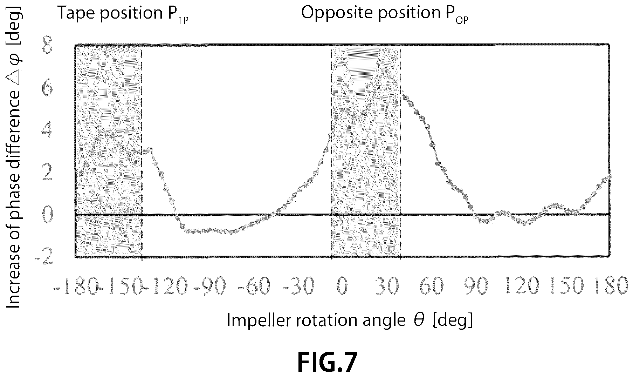

Fig. 7 is a characteristic diagram illustrating a change of a phase difference by the measured simulated thrombus (tape).

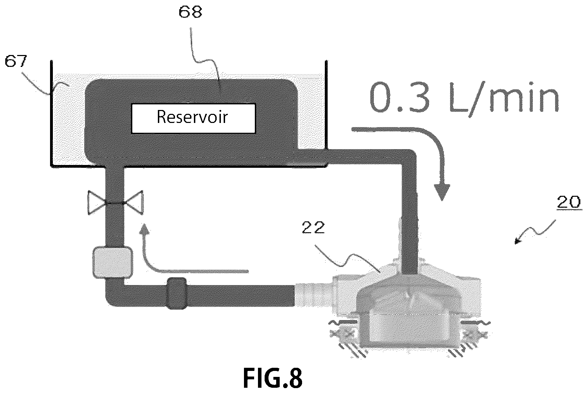

Fig. 8 is view schematically illustrating a structure of an experimental equipment for measuring a phase change when a thrombus is formed by using blood of a pig in the magnetically levitated blood pump system.

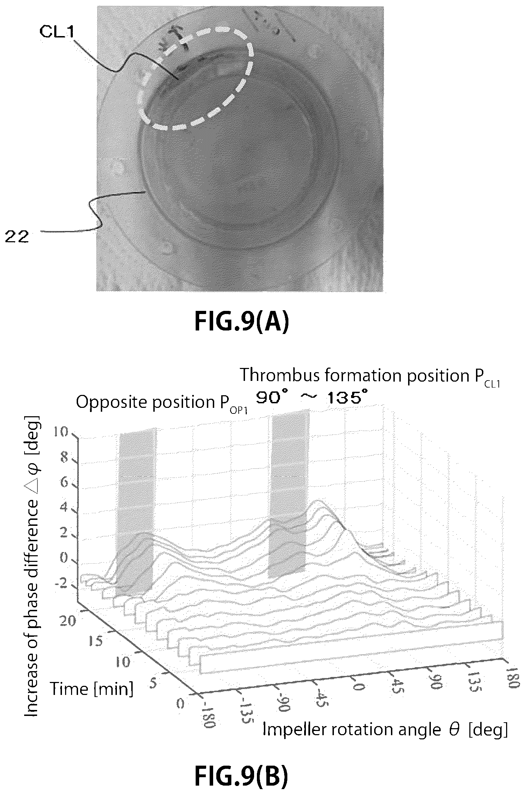

Fig. 9(A) and Fig. 9(B) are views illustrating a result of first experiment obtained by the experimental equipment, and Fig. 9(A) is illustrating an inner wall surface of the housing at which a thrombus is formed, and Fig. 9(B) is a characteristic diagram of a measured phase difference △cp.

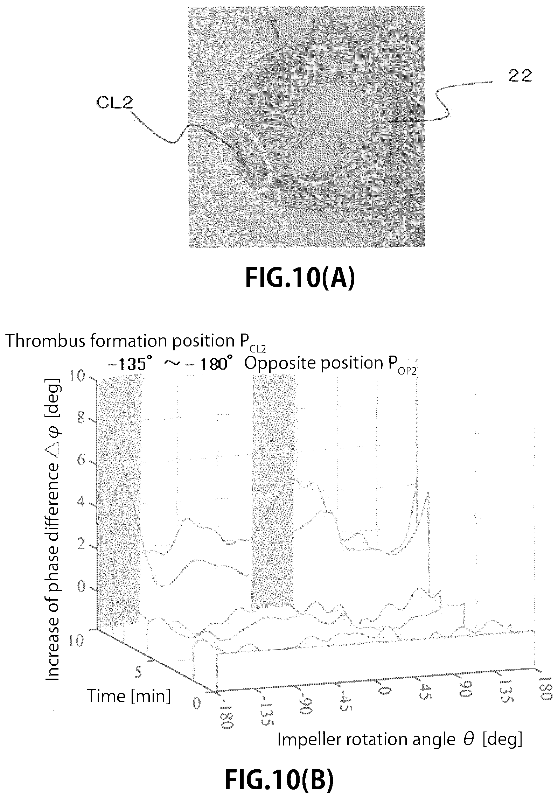

Fig. 10(A) and Fig. 10(B) are views illustrating a result of second experiment obtained by the experimental equipment, and Fig. 10(A) is illustrating an inner wall surface of the housing at which a thrombus is formed, and Fig. 10(B) is a characteristic diagram of a measured phase difference △cp.

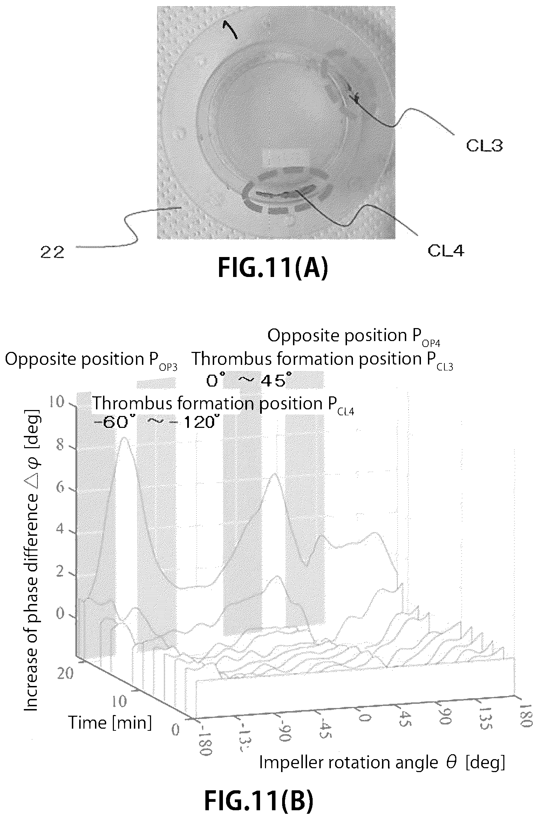

Fig. 11(A) and Fig. 11(B) are views illustrating a result of third experiment obtained by the experimental equipment, and Fig. 11(A) is illustrating an inner wall surface of the housing at which thrombi are formed, and Fig. 11(B) is a characteristic diagram of a measured phase difference △cp.

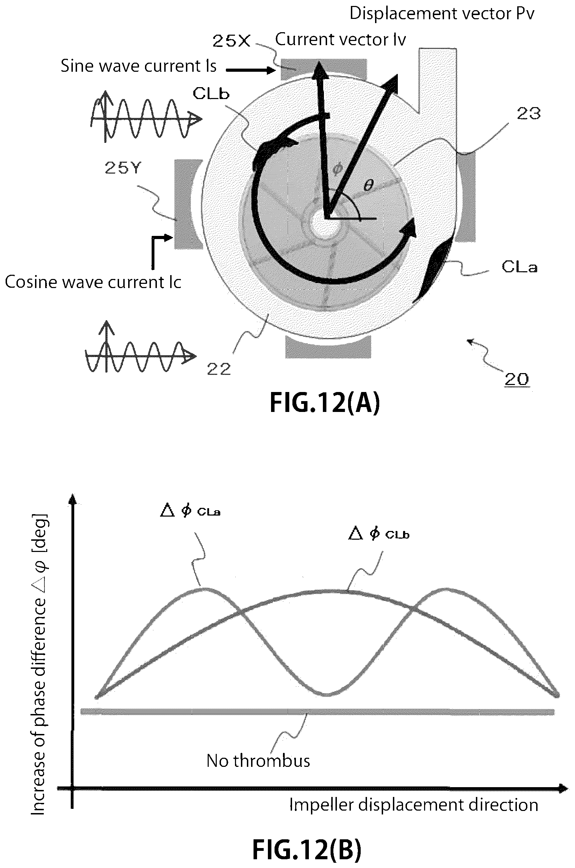

Fig. 12(A) and Fig. 12(B) are views for explaining about a detection of thrombus formed at a surface of the impeller in the magnetically levitated blood pump system, and Fig. 12(A) is a schematic plan view of the magnetically levitated blood pump illustrating a state that thrombi are formed respectively at an outer wall of the impeller and an inner wall of the housing, and Fig. 12(B) is a characteristic diagram of a phase difference △φ measured by rotating and revolving the impeller with a period of revolution double of a period of rotation.

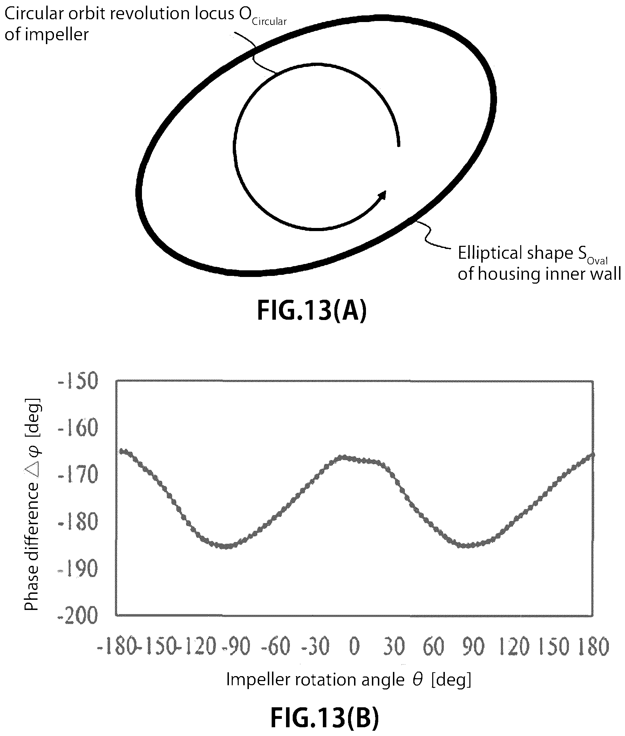

Fig. 13(A) and Fig. 13(B) are views for explaining a phase difference △φ measured when the housing is having an inner wall with an elliptical shape in the magnetically levitated blood pump system, and Fig. 13(A) is a view schematically illustrating an elliptical shape of the inner wall and a circular orbit of the revolving impeller, and Fig. 13(B) is a characteristic diagram of a measured phase difference △cp.

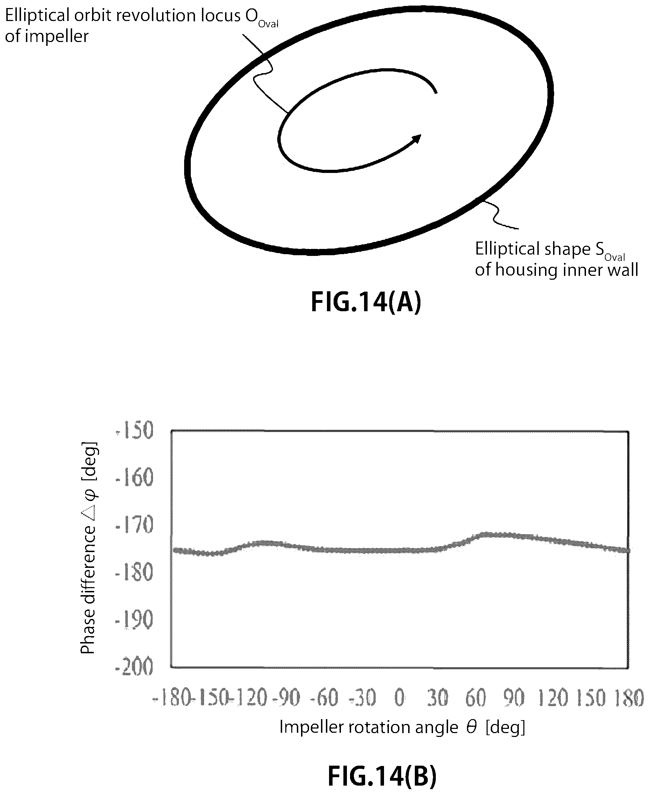

Fig. 14(A) and Fig. 14(B) are views for explaining a phase difference △φ measured when the housing is having an inner wall with an elliptical shape in the magnetically levitated blood pump system, and Fig. 14(A) is a view schematically illustrating an elliptical shape of the inner wall and an elliptical orbit of the revolving impeller, and Fig. 14(B) is a characteristic diagram of a measured phase difference △cp.

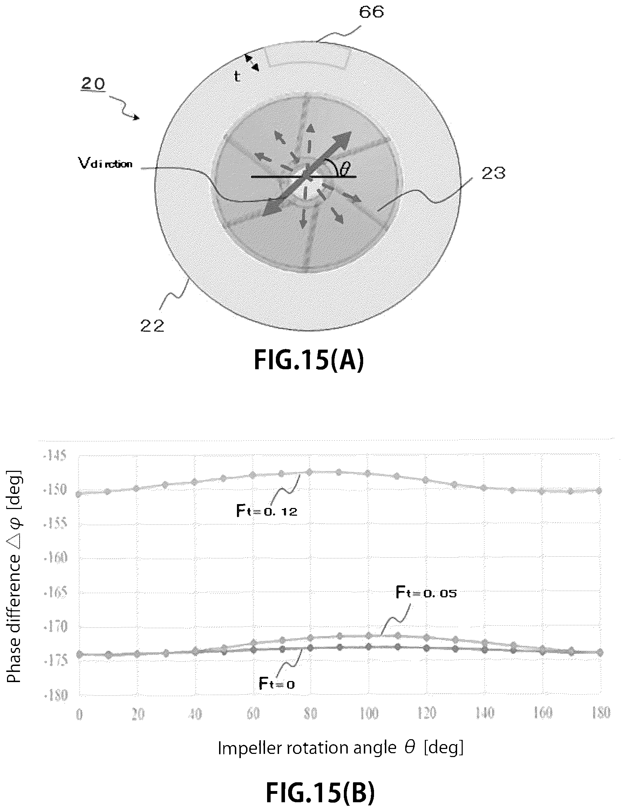

Fig. 15(A) and Fig. 15(B) are views for explaining a phase difference △φ measured when the impeller is revolved such that the impeller traces a revolving locus rotating an oscillation direction while simple oscillation of a rotation center of the impeller in a radial direction in the magnetically levitated blood pump system, and Fig. 15(A) is a schematic plan view of the magnetically levitated blood pump illustrating a revolving orbit of the impeller, and Fig. 15(B) is a characteristic diagram of a measured phase difference △cp.

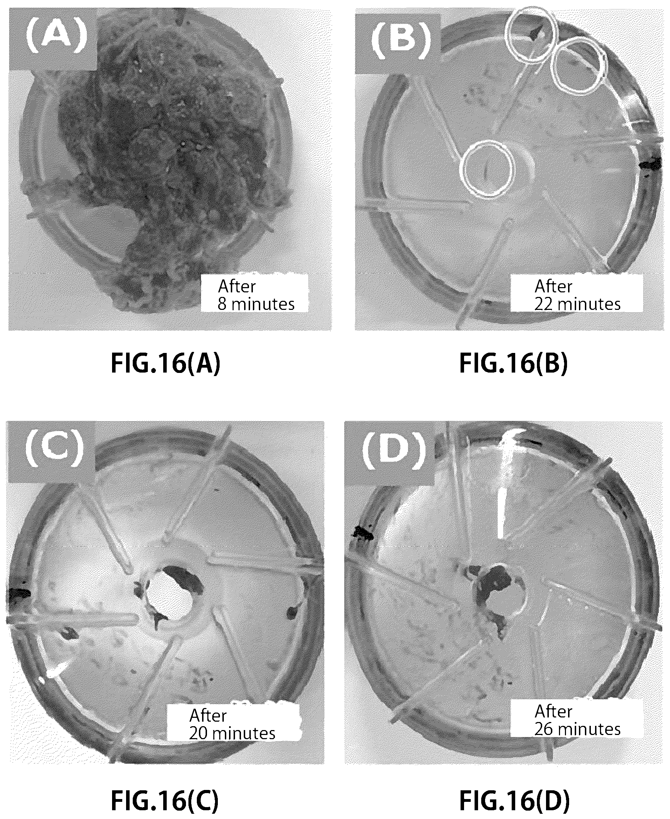

Fig. 16(A), Fig. 16(B), Fig. 16(C), and Fig. 16(D) are views for explaining an effect for preventing a formation of thrombus by a minute vibration of the impeller in the magnetically levitated blood pump system, and illustrating an observation result of a formation of thrombus obtained by measuring a thrombus formation time in the pump, wherein a frequency of a minute vibration of the impeller is f and an amplitude is a, Fig. 16(A) is illustrating thrombi formed when the impeller is not revolved, Fig. 16(B) is illustrating thrombi formed when the impeller is revolved with f=280Hz and a=3 $\mu$m (CCW), Fig. 16(C) is illustrating thrombi formed when the impeller is revolved with f=280Hz and a=3 $\mu$m (CW), and Fig. 16(D) is illustrating thrombi formed when the impeller is revolved with f=50Hz and a=16$\mu$m (CCW).

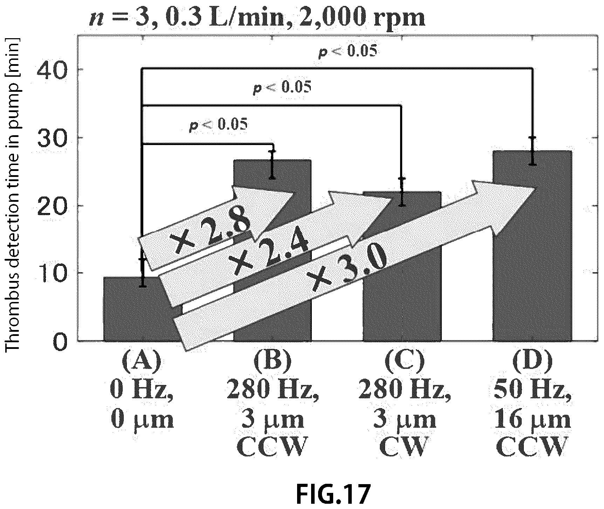

Fig. 17 is a view illustrating a magnification of a thrombus formation time in the pump respectively measured when the impeller is revolved with respect to a thrombus formation time in the pump measured when the impeller is not revolved.

Fig. 18(A) and Fig. 18(B) are views illustrating a structure of conventional centrifugal blood pumps used as an intracorporeal implanted blood pump (auxiliary artificial heart) used for assisting a blood circulation of a heart failure patient, and Fig. 18(A) is a partially sectional perspective view of a mechanical bearing type centrifugal blood pump having a contact bearing (pivot bearing), and Fig. 18(B) is a partially sectional perspective view of a magnetically levitated blood pump which does not use a mechanical bearing.

Fig. 19 is a plan view schematically illustrating a structure of a magnetically levitated blood pump for explaining a method for detecting a thrombus formed in the magnetically levitated blood pump previously reported in Non-Patent Literature 1 by the present inventors.

Detailed Description of the Invention

Hereinafter, it is explained about the preferred embodiments of the present invention. In addition, the present embodiments explained in below should not unjustly limit the content of the present invention described in claims, and not all of configurations explained in the present embodiments are necessary as a means for solving the problem of the present invention.

The present invention is performed, for example in an extracorporeal blood circulation system 10 with a structure as illustrated in Fig. 1.

Fig. 1 is a schematic view illustrating a structure of an extracorporeal blood circulation system 10 in which the present invention is performed.

This extracorporeal blood circulation system 10 comprises a centrifugal blood pump 20 comprising: a housing 22 having a blood inlet port 21A and a blood outlet port 21B connected to a blood supply tubes 11A, 11B; an impeller 23 for feeding blood by rotating in the housing 22; and an electric motor 24 for rotating the impeller 23, and this centrifugal blood pump 20 functions as an extracorporeal circulation path, in which a blood (vein blood) is extracted from an unillustrated human body via a blood inlet side blood supply tube 11A and extracted blood is returned to a vein or an artery of a patient via a blood outlet side blood supply tube 11B.

In a blood circulation path in the extracorporeal blood circulation system 10, a blood processing unit 15 for processing blood circulated via the blood circulation path can be arranged, and for example, an artificial lung for performing a gas exchange for taking in oxygen and discharging carbon dioxide in blood supplied via the blood outlet side blood supply tube 11B from the blood outlet port 21B of the centrifugal blood pump 20 can be arranged as the blood processing unit 15.

The centrifugal blood pump 20 in this extracorporeal blood circulation system 10 adopts a magnetically levitated blood pump comprising an electric motor for rotating the impeller 23 in the housing 22 by a magnetic coupling force in a Z axis direction in a state that the impeller 23 is magnetically levitated by electromagnets 25X, 25Y for magnetic levitation.

The centrifugal blood pump 20 in this extracorporeal blood circulation system 10 is drive controlled by a drive control device 30, and construct a magnetically levitated blood pump system 40 in which the impeller 23 is rotated and revolved by moving the impeller 23 to trace a revolving locus in which a rotation center of the impeller 23 in the housing 22 is moved in a radial direction.

The drive control device 30 in this magnetically levitated blood pump system 40 comprises a rotation drive control unit 31 and a revolution drive control unit 32 and control information is given to the drive control device 30 by a computational processing device 35.

The rotation drive control unit 31 functions as a rotation drive control means for rotating the impeller 23 by performing a drive control of the electric motor 24 for rotating the impeller 23 in the housing 22 with a magnetic coupling force based on control information given by the computational processing device 35.

Fig. 2 is a schematic plan view of the magnetically levitated blood pump 20 schematically illustrating a supply state of a revolution drive control current by the revolution drive control unit 32.

As illustrated in Fig. 2, the revolution drive control unit 32 functions as a revolution drive control means for revolving the impeller 23 in the housing 22 in a circular orbit by supplying a sine wave current Is and a cosine wave current Ic with a phase difference of 90 degrees as a revolution drive control current to electromagnets 25X and 25Y for magnetic levitation orthogonally arranged in a X axis direction and a Y axis direction orthogonal to the Z axis direction for magnetically levitating the impeller 23 in the housing 22.

In other words, this magnetically levitated blood pump system 40 rotates and revolves the impeller 23 in the housing 22 of the magnetically levitated blood pump 20 by the drive control device 30 comprising the rotation drive control unit 31 functioning as the rotation drive control means and the revolution drive control unit 32 functioning as the revolution drive control means for revolving the impeller 23 by moving the impeller 23 to trace a revolving locus in which a rotation center of the impeller 23 is moved in a radial direction.

Here, a structural example of the revolution drive control unit 32 in this magnetically levitated blood pump system 40 is illustrated in a block diagram of Fig. 3.

This revolution drive control unit 32 comprises a digital signal processor (DSP), and as its functional and structural example is illustrated in a block diagram of Fig. 3, a sine wave current XIsin and a cosine wave current YIcos are supplied to the electromagnets 25X and 25Y for magnetically levitating the impeller 23 in the housing 22 such that a rotation center position of the impeller 23 moves on a revolving orbit of a revolution radius designated by designation information $x_r$ for designating a rotation center position of the impeller 23 in a X axis direction and designation information $y_r$ for designating a rotation center position of the impeller 23 in a Y axis direction from a magnetic bearing controller 51, by giving the designation information $x_r$ and the designation information $y_r$ to a revolution drive circuit 50 consists of the digital signal processor (DSP) for maintaining a rotation center position of the impeller 23 in a state that the impeller 23 in the housing 22 is magnetically levitated.

In this revolution drive circuit 50, a signal waveform of a detection signal by a displacement sensor 27 for detecting an amount of displacement of a rotation center position of the impeller 23 is taken in via an A/D converter 52, and amplitudes Ax and Ay in a X axis direction and a Y axis direction of a displacement of a rotation center of the impeller 23 are respectively calculated by a Fourier transform unit 53. The amplitudes Ax and Ay obtained by the Fourier transform unit 53 fluctuates by an influence of a heartbeat or the like, so it is averaged by a low pass filter (LPF) 54. And, a difference between a target amplitude set by a target value setting unit 55 and an actual amplitude averaged by the low pass filter (LPF) 54 is input into a PID controller 56, and by multiplying its output with a sine wave signal ($\sin(2\pi ft)$) or a cosine wave signal ($\cos(2\pi ft)$), a target displacement ($x_r'$, $y_r'$) in a X axis direction and a Y axis direction of a rotation center of the impeller 23 is calculated, and the impeller 23 is excited and driven by inputting the target displacement into a magnetic bearing controller 51.

And, this revolution drive control unit 32 comprises a phase difference detection circuit 59 for calculating, by a phase calculator 58, an angle $\triangle\varphi$ formed by a current vector Iv and a displacement vector Pv expressing displacements x, y in a X axis direction and a Y axis direction of a rotation center of the impeller 23 by vectors by vector transform units 57P and 57I, by taking in a signal waveform of a sine wave current Is and a cosine wave current Ic supplied to the electromagnets 25X and 25Y for magnetic levitation from a current sensor 26 via the A/D converter 52, and also, by taking in a signal waveform of a detection signal by displacement sensors 27X and 27Y for detecting an amount of displacement of a rotation center position of the impeller 23 reciprocally moved in a X axis direction according to the sine wave current Is.

In other words, this phase difference detection circuit 59 calculates an angle (magnitude of phase delay) $\triangle\varphi$ formed by a current vector Iv and a displacement vector Pv by calculating a current vector Iv and a displacement vector Pv on a XY plane by measuring displacements x, y of the impeller 23 by a revolution of the impeller 23 and by measuring electromagnet currents Ix, Iy when revolving.

The angle (magnitude of phase delay) $\triangle\varphi$ formed by a current vector Iv and a displacement vector Pv changes inversely proportional to a cube of a gap $g_r$ with respect to an inner wall of the housing 22 or an outer wall of the impeller 23, but a change of a phase difference $\triangle\varphi$ depending on a viscosity $\mu$ of blood changes uniformly in all rotation angles of the impeller 23, and a change of a phase difference $\triangle\varphi$ depending on a change of the gap $g_r$ by a formation of thrombus changes according to a position of formation of thrombus.

Fig. 4(A) and Fig. 4(B) are views for explaining about a detection of thrombus CL by an excitation in a circular orbit of an impeller 23 in the magnetically levitated blood pump 20, and Fig. 4(A) is a schematic plan view of the magnetically levitated blood pump 20 illustrating a relative angle $\varphi$ between a displacement vector Pv of the impeller according to a revolution of the impeller 23 and a current vector Iv according to a revolution of the impeller 23, i.e. a phase of a drive current for revolving the impeller 23 in a circular orbit, and Fig. 4(B) is a characteristic diagram illustrating a change of a phase difference $\triangle cp$ by a formation of thrombus CL and a change of a phase difference $\triangle cp$ by a change of viscosity obtained by measuring the relative angle $\varphi$ between the displacement vector Pv of the impeller and the current vector Iv as a phase difference $\triangle cp$ between a phase of the drive current and a phase of a rotation of the impeller 23, as change features F1 and F2 according to a rotation angle $\theta$ of the impeller 23.

In other words, in a magnetically levitated blood pump system 40 for rotating and revolving the impeller 23 in the housing 22 of the magnetically levitated blood pump 20, as illustrated in Fig. 4(A), the impeller 23 is revolved in a circular orbit by supplying a sine wave current XIsin and a cosine wave current YIcos to electromagnets 25X and 25Y for magnetic levitation, so when measuring a relative angle $\varphi$ between a displacement vector Pv according to a rotation angle $\theta$ of the impeller 23 revolving in a circular orbit and a current vector Iv according to the revolution, as illustrated as a change feature F2 in Fig. 4(B), a change of a phase difference $\triangle\varphi$ depending on a viscosity $\mu$ of blood does not change by a rotation angle of the impeller 23, and as illustrated as a change feature F1 in Fig. 4(B), a change of a phase difference $\triangle\varphi$ depending on a change of gap $g_r$ by a formation of thrombus changes according to a position of a formation of thrombus CL. Therefore, a change of a viscosity $\mu$ of

blood and a formation of thrombus can be distinguished, and a position of a formation of thrombus CL can be detected.

Therefore, in this magnetically levitated blood pump system 40, based on a phase difference $\triangle\varphi$ obtained by a phase difference detection circuit 59 of the revolution drive control unit 32, a change of a viscosity $\mu$ of blood and a formation of thrombus can be distinguished, and a position of a formation of thrombus CL can be detected.

Here, Fig. 5(A) and Fig. 5(B) are views for explaining a change of a phase difference when a viscosity is changed in the magnetically levitated blood pump system 40, and Fig. 5(A) is a view schematically illustrating a structure of an experimental equipment for measuring a change of a phase difference when a viscosity is changed, and Fig. 5(B) is a characteristic diagram illustrating a change of measured phase difference.

In other words, in the magnetically levitated blood pump system 40, as illustrated in Fig. 5(A), when a phase difference $\triangle\varphi$ is measured until a final concentration of 12 vol% while administering 5.5 mL of glycerin at a charging speed of 1 mL/min by a syringe pump 65, as illustrated in Fig. 5(B), it was confirmed that a phase difference $\triangle\varphi$ is increased at all rotation angles of the impeller.

Also, Fig. 6(A) and Fig. 6(B) are views for explaining a change of a phase difference by a simulated thrombus (tape) about a detection of thrombus in the magnetically levitated blood pump system 40, and Fig. 6(A) is an exploded perspective view of a housing 22 of a magnetically levitated blood pump 20, and Fig. 6(B) is a view illustrating a state that a tape 66 is adhered as a substitute for thrombus at an inner wall of the housing 22. Fig. 7 is a characteristic diagram illustrating a change of a phase difference by the measured simulated thrombus (tape).

In other words, in the magnetically levitated blood pump system 40, as illustrated in Fig. 6(B), when a phase difference $\triangle\varphi$ is measured by adhering a tape 66 as a substitute for thrombus at an inner wall surface of a housing 22 of a magnetically levitated blood pump 20 as illustrated in an exploded perspective view of Fig. 6(A), as illustrated in Fig. 7, it was confirmed that a phase difference $\triangle\varphi$ is increased at a tape position $P_{TP}$ and its opposite position $P_{OP}$, and that a phase difference $\triangle cp$ is decreased at other positions. Therefore, by measuring a phase difference $\triangle\varphi$, a formation of thrombus can be detected and distinguished form a change of viscosity, and a position of thrombus can be estimated.

Fig. 8 is view schematically illustrating a structure of an experimental equipment for measuring a phase change when a thrombus is formed by using blood of a pig in the magnetically levitated blood pump system 40.

In other words, in the magnetically levitated blood pump system 40, as illustrated in Fig. 8, when experiments were performed to measure a phase change when a thrombus is formed by using blood of a pig, results of experiments were obtained as illustrated in Fig. 9(A) and Fig. 9(B) to Fig. 1 1(A) and Fig. 1 1(B), and it was confirmed that a position of thrombus formed at an inner wall surface of the housing 22 of the magnetically levitated blood pump 20 can be detected.

The experiments were performed three times respectively as illustrated in Fig. 8, in a state that blood in the housing 22 is returned to a reservoir 68 by rotating the impeller 23 in the housing 22 at 2000 rpm while flowing blood of a pig into the housing 22 of the magnetically levitated blood pump 20 with a flow rate of 0.3L/min via the reservoir 68 maintained at 37 degrees Celsius by a thermostatic bath 67, a relative angle $\varphi$ between a current vector Iv according to a revolution of the impeller 23 and a displacement vector Pv according to a rotation angle $\theta$ of the impeller 23 was measured as a phase difference $\triangle\varphi$.

Fig. 9(A) and Fig. 9(B) are views illustrating a result of first experiment obtained by the experimental equipment, and Fig. 9(A) is illustrating an inner wall surface of the housing 22 at which a thrombus is formed, and Fig. 9(B) is a characteristic diagram of a measured phase difference $\triangle cp$.

In other words, in a first experiment, as illustrated in Fig. 9(A), a thrombus CL1 is formed at an inner wall surface of the housing 22 with a rotation angle $\theta$ of the impeller in a range of 90 to 135 degrees, and as illustrated in Fig. 9(B), a phase difference $\triangle\varphi$ at a position $P_{CL1}$ of thrombus formation with a rotation angle $\theta$ of the impeller of 90 to 135 degrees and its opposite position $P_{OP1}$ were increased as time passes.

Fig. 10(A) and Fig. 10(B) are views illustrating a result of second experiment obtained by the experimental equipment, and Fig. 10(A) is illustrating an inner wall surface of the housing 22 at which a thrombus is formed, and Fig. 10(B) is a characteristic diagram of a measured phase difference $\triangle\varphi$.

In other words, in a second experiment, as illustrated in Fig. 10(A), a thrombus CL2 is formed at an inner wall surface of the housing 22 with a rotation angle $\theta$ of the impeller in a range of -135 to -180 degrees, and as illustrated in Fig. 10(B), a phase difference $\triangle\varphi$ at a position $P_{CL2}$ of thrombus formation with a rotation angle $\theta$ of the impeller of -135 to -180 degrees and its opposite position $P_{OP2}$ were increased as time passes.

Fig. 11(A) and Fig. 11(B) are views illustrating a result of third experiment obtained by the experimental equipment, and Fig. 11(A) is illustrating an inner wall surface of the housing 22 at which thrombi are formed, and Fig. 11(B) is a characteristic diagram of a measured phase difference $\triangle cp$.

In other words, in a third experiment, as illustrated in Fig. 11(A), thrombi CL3 and CL4 are formed at an inner wall surface of the housing 22 with a rotation angle $\theta$ of the impeller respectively in a range of -60 to -120 degrees and 0 to 45 degrees, and as illustrated in Fig. 11(B), a phase difference $\triangle\varphi$ at positions $P_{CL3}$ and $P_{CL4}$ of respective thrombi formation with a rotation angle $\theta$ of the impeller of -60 to -120 degrees and 0 to 45 degrees and its opposite positions $P_{OP3}$ and $P_{OP4}$ were increased as time passes.

By the results of experiments for measuring a phase change when thrombus is formed by using blood of a pig, it was confirmed that a position of thrombus formed at an inner wall surface of the housing 22 can be detected.

Also, in the magnetically levitated blood pump system 40, the drive control device 30 rotates and revolves the impeller 23 in a period of revolution which is an integer multiple of a period of rotation T or an integer division of a period of rotation T, by a rotation drive control unit 31 functioning as a rotation drive control means and a revolution drive control unit 32 functioning as a revolution drive control means for revolving the impeller 23 by moving the impeller 23 to trace a revolving locus in which a rotation center of the impeller 23 is moved in a radial direction.

Here, Fig. 12(A) and Fig. 12(B) are views for explaining about a detection of thrombus formed at a surface of the impeller 23 in the magnetically levitated blood pump system 40, and Fig. 12(A) is a schematic plan view of the magnetically levitated blood pump 20 illustrating a state that thrombi CLa, CLb are formed respectively at an outer wall of the impeller 23 and an inner wall of the housing 22, and Fig. 12(B) is a characteristic diagram of a phase difference $\triangle\varphi$ measured by rotating and revolving the impeller 23 with a period of revolution double of a period of rotation.

In other words, in the magnetically levitated blood pump system 40, by rotating and revolving the impeller 23 with a period of revolution 2T double of a period of rotation T, for example as illustrated in Fig. 12(A), about a case that thrombi CLa, CLb are formed respectively at an outer wall of the impeller 23 and an inner wall of the housing 22, a result of measuring a relative angle $\varphi$ between a current vector Iv according to a revolution of the impeller 23 and a displacement vector Pv according to a rotation angle $\theta$ of the impeller 23 as a phase difference $\triangle$cp is illustrated in Fig. 12(B), and a phase difference $\triangle\varphi_{CLa}$ by a thrombus CLa formed at an inner wall of the housing 22 changes in a period double of a phase difference $\triangle\varphi_{CLb}$, by a thrombus CLb formed at an outer wall of the impeller 23, so the thrombus CLa formed at an inner wall of the housing 22 and the thrombus CLb formed at an outer wall of the impeller 23 can be detected and distinguished by a change of the phase difference $\triangle$cp.

In addition, a period of revolution of the impeller 23 is not limited to 2T which is a double of a period of rotation T, and by rotating and revolving the impeller 23 in a period of revolution which is an integer multiple of a period of rotation T or an integer division of a period of rotation T, by a revolution drive control unit 32 functioning as a revolution drive control means for revolving the impeller 23, the thrombus CLa formed at an inner wall of the housing 22 and the thrombus CLb formed at an outer wall of the impeller 23 can be detected and distinguished from a change of a phase difference $\triangle$cp by

detecting a relative angle $\varphi$ between a current vector Iv according to a revolution of the impeller 23 and a displacement vector Pv according to a rotation angle $\theta$ of the impeller 23 as the phase difference $\triangle$cp.

Also, in the drive control device 30 in the magnetically levitated blood pump system 40, the impeller 23 is revolved in a circular orbit by the revolution drive control unit 32, but an orbit of the impeller 23 is not limited to a circular orbit, and the impeller 23 may be revolved in an elliptical orbit, or the impeller 23 may be revolved to trace a revolving locus rotating an oscillation direction while simple oscillation of a rotation center of the impeller 23 in a radial direction.

Here, Fig. 13(A) and Fig. 13(B) are views for explaining a phase difference $\triangle$cp measured when the housing 22 is having an inner wall with an elliptical shape $S_{Oval}$ in the magnetically levitated blood pump system 40, and Fig. 13(A) is a view schematically illustrating an elliptical shape $S_{Oval}$ of the inner wall and a circular orbit $O_{Circular}$ of the revolving impeller 23, and Fig. 13(B) is a characteristic diagram of a measured phase difference $\triangle$cp.

In other words, as illustrated in Fig. 13(A), when an inner wall of the housing 22 of the magnetically levitated blood pump 20 is having an elliptical shape $S_{Oval}$ and not a perfect circle by a machining error or the like, and an orbit of the impeller 23 is in a circular orbit $O_{Circular}$, a relative angle $\varphi$ between a current vector Iv according to a revolution of the impeller 23 and a displacement vector Pv according to a rotation angle $\theta$ of the impeller 23 is measured as a phase difference $\triangle$cp, and as illustrated in Fig. 13(B), a phase difference $\triangle\varphi$ according to a rotation angle $\theta$ of the impeller 23 will be uneven, even when a thrombus is not formed.

Fig. 14(A) and Fig. 14(B) are views for explaining a phase difference $\triangle\varphi$ measured when the housing 22 is having an inner wall with an elliptical shape $S_{Oval}$ in the magnetically levitated blood pump system 40, and Fig. 14(A) is a view schematically illustrating an elliptical shape $S_{Oval}$ of the inner wall and an elliptical orbit $O_{Oval}$ of the revolving impeller 23, and Fig. 14(B) is a characteristic diagram of a measured phase difference $\triangle$cp.

In other words, the revolution drive control unit 32 of the drive control device 30 can revolve the impeller 23 in the housing 22 in an elliptical orbit $O_{Oval}$ by deviating a phase of one of a sine wave current XIsin and a cosine wave current Ic to be supplied to electromagnets 25X, 25Y for magnetically levitating the impeller 23 in the housing 22. By adjusting an amount of deviation of a phase of one of a sine wave current XIsin and a cosine wave current Ic to be supplied to the electromagnets 25X, 25Y for magnetic levitation, as illustrated in Fig. 14(A), the impeller 23 can be revolved in an elliptical orbit $O_{Oval}$ corresponding to an elliptical shape $S_{Oval}$ of an inner wall of the housing 22, and when a relative angle $\varphi$ between a current vector Iv according to a revolution of the impeller 23 and a displacement vector Pv according to a rotation angle $\theta$ of the impeller 23 is measured as a phase difference Δcp by revolving the impeller 23 in such elliptical orbit, as illustrated in Fig. 14(B), an unevenness of a phase difference △φ according to a rotation angle θ of the impeller 23 occurred in a revolution of a circular orbit $O_{Circular}$ can be resolved.

Also, by sequentially changing an amount of deviation of a phase of one of a sine wave current XIsin and a cosine wave current Ic supplied to the electromagnets 25X, 25Y for magnetic levitation, as illustrated in Fig. 15(A), the impeller 23 can be revolved to trace a revolving locus rotating an oscillation direction while simple oscillation of a rotation center of the impeller 23 in a radial direction.

Here, Fig. 15(A) and Fig. 15(B) are views for explaining a phase difference Δcp measured when the impeller 23 is revolved such that the impeller 23 traces a revolving locus rotating an oscillation direction $V_{direction}$ while simple oscillation of a rotation center of the impeller 23 in a radial direction in the magnetically levitated blood pump system 40, and Fig. 15(A) is a schematic plan view of the magnetically levitated blood pump 20 illustrating an oscillation direction $V_{direction}$ of the impeller 23, and Fig. 15(B) is a characteristic diagram of a measured phase difference Δcp.

In a state that the impeller 23 is revolved such that the impeller 23 traces a revolving locus rotating an oscillation direction $V_{direction}$ while simple oscillation of a rotation center of the impeller 23 in a radial direction and that a tape with a thickness t is adhered to an inner wall of the housing 22, a relative angle φ between a current vector Iv according to a revolution of the impeller 23 and a displacement vector Pv according to a rotation angle θ of the impeller 23 is measured as a phase difference Δcp, and as illustrated in Fig. 15(B), in a phase difference feature $F_{t=0}$ without a tape, i.e. t=0, a phase difference △φ according to a rotation angle θ of the impeller 23 is not changed, but in a phase difference feature $F_{t=0.05}$ in which a tape of t=0.05 mm is adhered, and in a phase difference feature $F_{t=0.12}$ in which a tape of t=0.12 mm is adhered, a phase difference Δcp is increased at a position of a rotation angle of the impeller 23 according to an adhered position of the tape.

Therefore, by revolving the impeller 23 such that the impeller 23 traces a revolving locus rotating an oscillation direction $V_{direction}$ while simple oscillation of a rotation center of the impeller 23 in a radial direction, a position of thrombus formed at an inner wall of the housing 22 can be estimated based on a measurement result of the phase difference Δcp.

Further, in the magnetically levitated blood pump system 40 drive controlled by the drive control device 30 as the above to rotate and revolve the impeller 23 by moving the impeller 23 to trace a revolving locus in which a rotation center of the impeller 23 in the housing 22 is moved in a radial direction, the impeller 23 in the housing 22 is vibrated minutely in a radial direction by a revolution, so a formation of thrombus can be prevented by preventing an adhesion of thrombocyte to the impeller 23 by a minute vibration of the impeller 23.

In other words, in the magnetically levitated blood pump system 40, by performing an experiment to measure a thrombus formation time in a pump based on a phase change when a thrombus is formed by using blood of the pig, an effect for preventing a formation of thrombus by a minute vibration of the impeller 23 was confirmed as below.

Here, Fig. 16(A), Fig. 16(B), Fig. 16(C), and Fig. 16(D) are views for explaining an effect for preventing a formation of thrombus by a minute vibration of the impeller 23 in the magnetically levitated blood pump system 40, and illustrating an observation result of a formation of thrombus obtained by measuring a thrombus formation time in the pump, wherein a frequency of a minute vibration of the impeller 23 is f and an amplitude is a, Fig. 16(A) is illustrating thrombi formed when the impeller 23 is not revolved with a thrombus formation time in the pump of 8 minutes, Fig. 16(B) is illustrating thrombi formed when the impeller 23 is revolved with f=280Hz and a=3μm (CCW) with a thrombus formation time in the pump of 22 minutes, Fig. 16(C) is illustrating thrombi formed when the impeller 23 is revolved with f=280Hz and a=3μm (CW) with a thrombus formation time in the pump of 20 minutes, and Fig. 16(D) is illustrating thrombi formed when the impeller 23 is revolved with f=50Hz and a=16μm (CCW) with a thrombus formation time in the pump of 26 minutes. Also, Fig. 17 is a view illustrating a magnification of a thrombus formation time in the pump respectively measured when the impeller 23 is revolved with respect to a thrombus formation time in the pump measured when the impeller 23 is not revolved.

When the impeller 23 was not revolved, i.e. f=0Hz and a=0μm, as illustrated in Fig. 16(A), a formation of a large quantity of thrombi were observed with a thrombus formation time in the pump of 8 minutes, on the other hand, when the impeller 23 was revolved with f=280Hz and a=3μm (CCW), as illustrated in Fig. 16(B), a formation of thrombi were observed with a thrombus formation time in the pump of 22 minutes, and also, when the impeller 23 was revolved with f=280Hz and a=3μm (CW), as illustrated in Fig. 16(C), a formation of thrombi were observed with a thrombus formation time in the pump of 20 minutes, and further, when the impeller 23 was revolved with f=50Hz and a=16μm (CCW), as illustrated in Fig. 16(D), a formation of thrombi were observed with a thrombus formation time in the pump of 26 minutes, and as illustrated in Fig. 17, with respect to a thrombus formation time in the pump of 8 minutes when the impeller 23 was not revolved, a thrombus formation time in the pump of 22 minutes is 2.8 times thereof, a thrombus formation time in the pump of 20 minutes is 2.4 times thereof, and a thrombus formation time in the pump of 26 minutes is 3 times thereof, and in all cases, $p < 0.05$, i.e. adopt a significance level of 5% at both sides, and there is a statistic significant difference, so an effect for preventing a formation of thrombus by a minute vibration of the impeller 23 was confirmed.

Glossary of Drawing References

10 Extracorporeal blood circulation system
11A Blood inlet side blood supply tube
11B Blood outlet side blood supply tube
15 Blood processing unit
20 Magnetically levitated blood pump
21A Blood inlet port
21B Blood outlet port
22 Housing
23 Impeller
24 Electric motor
25X, 25Y Electromagnets for magnetic levitation
26 Current sensor
27X, 27Y Displacement sensor
30 Drive control device
31 Rotation drive control unit
32 Revolution drive control unit
35 Computational processing device
40 Magnetically levitated blood pump system
50 Revolution drive circuit
51 Magnetic bearing controller
52 A/D converter
53 Fourier transform unit
54 Low pass filter
55 Target value setting unit
56 PID controller
57P, 57I Vector transform unit
58 Phase calculator
59 Phase difference detection circuit
65 Syringe pump
66 Tape
CL1 to CL4, CLa, CLb Thrombus
$P_{CL1}$ to $P_{CL4}$ Position of thrombus formation
$P_{OP1}$ to $P_{OP4}$ Opposite position

**Claims**

1. A drive control method for a magnetically levitated blood pump for rotating an impeller in a housing in a state magnetically levitated by electromagnets, wherein the impeller is rotated and revolved by moving the impeller to trace a revolving locus in which a rotation center of the impeller is moved in a radial direction.

2. The drive control method for a magnetically levitated blood pump according to claim 1, wherein a period of revolution of the impeller is an integer multiple of a period of rotation of the impeller or an integer division of a period of rotation of the impeller.

3. The drive control method for a magnetically levitated blood pump according to claim 1 or 2, wherein the impeller is revolved in a circular orbit.

4. The drive control method for a magnetically levitated blood pump according to claim 1 or 2, wherein the impeller is revolved in an elliptical orbit.

5. The drive control method for a magnetically levitated blood pump according to claim 1 or 2, wherein the impeller is revolved to trace a revolving locus rotating an oscillation direction while simple oscillation of a rotation center of the impeller in a radial direction.

6. The drive control method for a magnetically levitated blood pump according to claim 3, wherein the impeller in the housing is revolved in a circular orbit by supplying a sine wave current and a cosine wave current to electromagnets for magnetic levitation orthogonally arranged in a X axis direction and a Y axis direction orthogonal to a rotation axis direction of the impeller for magnetically levitating the impeller in the housing, while rotating the impeller in the housing by an electric motor with a magnetic coupling force.

7. The drive control method for a magnetically levitated blood pump according to claim 4, wherein the impeller in the housing is revolved in an elliptical orbit by deviating a phase of one of a sine wave current and a cosine wave current supplied to electromagnets for magnetic levitation orthogonally arranged in a X axis direction and a Y axis direction orthogonal to a rotation axis direction of the impeller for magnetically levitating the impeller in the housing, while rotating the impeller in the housing by an electric motor with a magnetic coupling force.

8. A drive control device for a magnetically levitated blood pump for rotating an impeller in a housing in a state magnetically levitated by electromagnets, comprising:

    a rotation drive control means for rotating the impeller by an electric motor with a magnetic coupling force; and
    a revolution drive control means for revolving the impeller by moving the impeller to trace a revolving locus in which a rotation center of the impeller is moved in a radial direction,
    wherein the impeller is rotated and revolved.

9. The drive control device for a magnetically levitated blood pump according to claim 8, wherein the rotation drive control means and the revolution drive control means rotate and revolve the impeller in a period of revolution which is an integer multiple of a period of rotation or an integer division of a period of rotation.

10. The drive control device for a magnetically levitated blood pump according to claim 8 or 9, wherein the revolution drive control means revolves the impeller in a circular orbit.

11. The drive control device for a magnetically levitated blood pump according to claim 8 or 9, wherein the revolution drive control means revolves the impeller in an elliptical orbit.

12. The drive control device for a magnetically levitated blood pump according to claim 8 or 9, wherein the revolution drive control means revolves the impeller to trace a revolving locus rotating an oscillation direction while simple oscillation of a rotation center of the impeller in a radial direction.

13. The drive control device for a magnetically levitated blood pump according to claim 10, wherein the rotation drive control means rotates the impeller in the housing by an electric motor with a magnetic coupling force, and
the revolution drive control means revolves the impeller in the housing in a circular orbit by supplying a sine wave current and a cosine wave current to electromagnets for magnetic levitation orthogonally arranged in a X axis direction and a Y axis direction orthogonal to a rotation axis direction of the impeller for magnetically levitating the impeller in the housing.

14. The drive control device for a magnetically levitated blood pump according to claim 13, wherein the revolution drive control means revolves the impeller in the housing in an elliptical orbit by deviating a phase of one of the sine wave current and the cosine wave current supplied to the electromagnets for magnetically levitating the impeller in the housing.

15. A magnetically levitated blood pump system, comprising:

the drive control device according to any of claims 8 to 14; and
a magnetically levitated blood pump which is drive controlled by the drive control device,
wherein an impeller of the magnetically levitated blood pump is rotated and revolved.

16. The magnetically levitated blood pump according to claim 15, further comprising a computing means for calculating a phase difference between a locus of displacement of the impeller when the impeller is revolved and a locus of current of electromagnets for revolving the impeller.

FIG.1

**FIG.2**

**FIG.3**

FIG.4(A)

FIG.4(B)

**FIG.5(A)**

Impeller rotation angle $\theta$ [deg]

Increase of phase difference $\triangle \varphi$ [deg]

**FIG.5(B)**

**FIG.6(A)**

Opposite side

**FIG.6(B)**

FIG.7

FIG.8

**FIG.9(A)**

**FIG.9(B)**

**FIG.10(A)**

**FIG.10(B)**

**FIG.11(A)**

**FIG.11(B)**

Displacement vector Pv

25X — Current vector Iv

Sine wave current Is ——→

CLb

25Y

23

Cosine wave current Ic

$\phi$

$\theta$

CLa

22

20

**FIG.12(A)**

Increase of phase difference $\triangle \varphi$ [deg]

$\triangle \phi$ CLa

$\triangle \phi$ CLb

No thrombus

Impeller displacement direction

**FIG.12(B)**

Circular orbit revolution locus $O_{Circular}$ of impeller

Elliptical shape $S_{Oval}$ of housing inner wall

**FIG.13(A)**

Phase difference $\triangle \varphi$ [deg]

Impeller rotation angle $\theta$ [deg]

**FIG.13(B)**

Elliptical orbit revolution locus $O_{Oval}$
of impeller

Elliptical shape $S_{Oval}$
of housing inner wall

## FIG.14(A)

Phase difference $\triangle \varphi$ [deg]

-150
-160
-170
-180
-190
-200

-180 -150 -120 -90 -60 -30 0 30 60 90 120 150 180

Impeller rotation angle $\theta$ [deg]

## FIG.14(B)

FIG.15(A)

Impeller rotation angle $\theta$ [deg]

FIG.15(B)

FIG.16(A)

FIG.16(B)

FIG.16(C)

FIG.16(D)

FIG.17

FIG.18(A)

FIG.18(B)

200

202X

Sine wave current Is

202Y

Direct current Idc

205

207Y

Sy

201

Sx

207X

**FIG.19**

## INTERNATIONAL SEARCH REPORT

| International application No. |
|---|
| **PCT/JP2023/006133** |

### A. CLASSIFICATION OF SUBJECT MATTER

*A61M 60/822*(2021.01)i; *A61M 60/113*(2021.01)i; *A61M 60/117*(2021.01)i; *A61M 60/232*(2021.01)i;
*A61M 60/38*(2021.01)i; *A61M 60/419*(2021.01)i; *A61M 60/508*(2021.01)i; *A61M 60/804*(2021.01)i; *F04D 29/048*(2006.01)i;
*F16C 32/04*(2006.01)i
FI: A61M60/822; A61M60/113; A61M60/117; A61M60/232; A61M60/38; A61M60/419; A61M60/508; A61M60/804;
F16C32/04 A; F04D29/048

According to International Patent Classification (IPC) or to both national classification and IPC

### B. FIELDS SEARCHED

Minimum documentation searched (classification system followed by classification symbols)

A61M60/822; A61M60/113; A61M60/117; A61M60/232; A61M60/38; A61M60/419; A61M60/508; A61M60/804;
F04D29/048; F16C32/04

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2007-044302 A (NATIONAL UNIVERSITY CORPORATION TOKYO MEDICAL AND DENTAL UNIVERSITY) 22 February 2007 (2007-02-22) paragraphs [0029]-[0032], fig. 4 | 1, 8, 15 |
| A | | 2-7, 9-14, 16 |
| A | JP 2002-345787 A (INSTITUTE OF TSUKUBA LIAISON CO LTD) 03 December 2002 (2002-12-03) | 1-16 |
| A | JP 63-263290 A (FUKUI, Yasuhiro) 31 October 1988 (1988-10-31) | 1-16 |
| A | JP 2020-139484 A (UNIV SAITAMA) 03 September 2020 (2020-09-03) | 1-16 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **11 April 2023** | **25 April 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/006133**

| Patent document cited in search report | | | Publication date (day/month/year) | Patent family member(s) | | | Publication date (day/month/year) |
|---|---|---|---|---|---|---|---|
| JP | 2007-044302 | A | 22 February 2007 | US | 2009/0118625 | A1 | |
| | | | | paragraphs [0028]-[0031], fig. 4 | | | |
| | | | | EP | 1913963 | A1 | |
| | | | | WO | 2007/018044 | A1 | |
| JP | 2002-345787 | A | 03 December 2002 | (Family: none) | | | |
| JP | 63-263290 | A | 31 October 1988 | US | 4869650 | A | |
| | | | | EP | 287924 | A2 | |
| JP | 2020-139484 | A | 03 September 2020 | (Family: none) | | | |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009106690 A
- JP 2020139484 A

- WO 2010104031 A1

**Non-patent literature cited in the description**

- **WATARU H** ; **TAKURO M** ; **TOMOTAKA M** ; **DAISUKE S** ; **OSAMU M**. Detection of thrombosis in a magnetically levitated blood pump by vibrational excitation of the impeller. *Artificial Organs*, 2020, vol. 44, 594-603